# EUROPEAN PATENT SPECIFICATION

(11) **EP 4 277 719 B1**
(45) Date of publication and mention of the grant of the patent: **09.09.2026**
(21) Application number: 22700767.1
(22) Date of filing: 12.01.2022
(51) Int. Cl.: B01D 3/00, B01D 3/14, B01D 53/14, C01B 32/50, C07C 7/04, C07C 29/80, C10K 1/00, C10K 1/08, F25J 3/02

(54) **METHOD FOR PRODUCTION OF METHANOL**
VERFAHREN ZUR PRODUKTION VON METHANOL
PROCÉDÉ DE PRODUCTION DE MÉTHANOL

(30) Priority: 13.01.2021 EP 21151426
(43) Date of publication of application: 22.11.2023
(73) Proprietor: Topsoe A/S, 2800 Kgs. Lyngby (DK)
(72) Inventor: TJÄRNEHOV, Emil Andreas, 216 22 Limhamn (SE)
(74) Representative: Topsoe A/S
(86) International application number: PCT/EP2022/050541
(87) International publication number: WO 2022/152749

(56) References cited:
- WO-A1-2019/232626
- US-A- 5 346 593
- US-A1- 2011 201 700
- US-A1- 2015 353 454

## Description

### Field of Invention

The present invention refers to a method for the reduction of energy consumption in a green methanol plant.

### Background of Invention

It is known that the product of plants for synthesizing methanol, commonly defined as crude methanol, is an aqueous solution of methanol containing by-products of the synthesis reaction including ethanol, ketones, higher alcohols, and some dissolved gases including mainly H₂, CO, CO₂, N₂, CH₄.

Known distillation processes are based substantially on one or more distillation columns, where typically at least one column is able to separate light products (for example gas) recovered at the top of the column from methanol, and at least one column is able to separate the heavier product (e.g. aqueous solution) recovered at the bottom of the column from methanol.

A specific method which is widely used for e.g. distillation of methanol, comprises two columns that operate at atmospheric pressure or close to atmospheric pressure. More specifically, said method uses a preliminary treatment column known as stabilizing column or pre-run column and a second distillation column. The first column substantially has the purpose of separating the more volatile components contained in the crude methanol, where it receives the crude methanol and separates the light components at the top and an aqueous solution at the bottom. The second column known as concentration column carries out the actual distillation, obtaining (i) refined methanol at the top, (ii) a prevalently aqueous stream at the bottom ("bottom water"), (iii) a side stream known as "fusel oil" mainly containing water, residual methanol (ca. 1 % of the total) and most of the by-products of the synthesis reaction. Said fusel oil has a certain heat value and is usually used as a fuel or feed in for synthesis gas generation.

Each column comprises a reboiler that heats the bottom of the column and maintains heat input to the distillation method. Each column comprises also a condenser, which condenses the top product and recycles it (at least partially) to said column. The heat is provided to the concentration (or distillation) column by steam, or by a process gas - when available - of suitable thermal level. The cooling medium for the condenser is normally water or air. Said configuration with two columns is simple in terms of a plant (e.g., a methanol distillation plant), but it has the major drawback of consuming a substantial amount of energy, both due to the heat supplied to the bottom reboilers, and due to the consumption of cooling water and/or electricity of the top condensers. Moreover, the columns have a relatively large diameter in relation to the production capacity and the plant cost is consequently high.

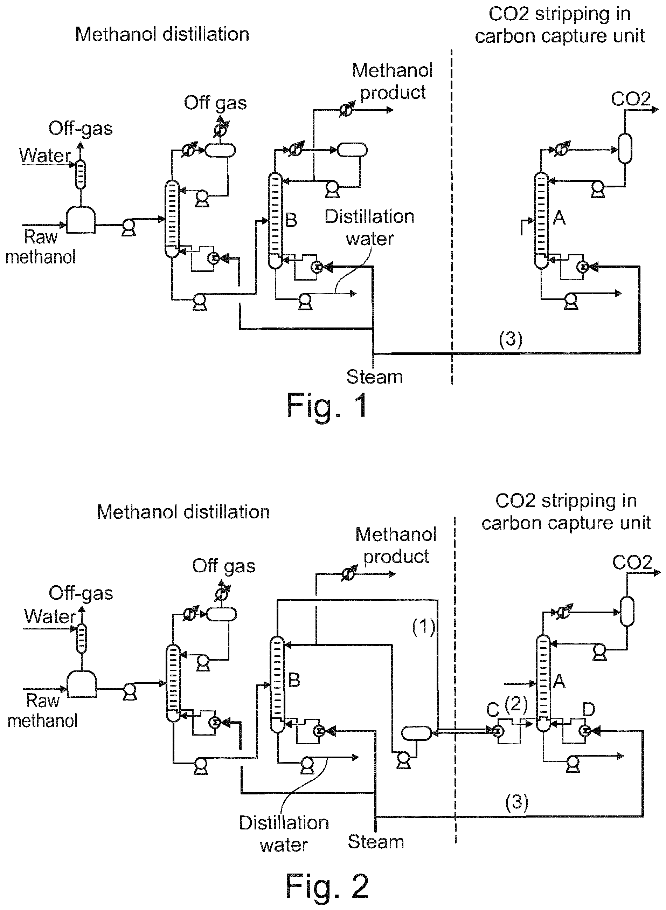

The standard solution shown in Figure 1 is based on a low pressure concentration column and the methanol vapor generated is condensed by a water cooler. For larger plants the concentration column is split into two or three distillation columns with staggered pressure levels in order to re-use the overhead duty as reboiler duty in the next column. Typically, the required heat for methanol distillation and CO₂ stripping is provided mainly or totally by steam. An example of carbon capture and a methanol production system is provided in US2015/353454.

There is, however, the need for the pursuit of more sustainable ways of working in methanol production, providing for the reutilization and/or saving of energy.

The combustion of fossil fuels generates over 13 gigatons of CO₂ per year. Concern over the effects of CO₂ with respect to climate change and ocean acidification led governments and industries to investigate the feasibility of technologies that capture the resultant CO₂ from entering the carbon cycle. However, existing power plants require the post-combustion separation of CO₂ from the flue gas with a scrubber. In such a system, fossil fuels are combusted with air and CO₂ is selectively removed from a gas mixture also containing N₂, H₂O, O₂ and trace sulphur, nitrogen and metal impurities. While exact separation conditions are fuel and technology dependent, in general CO₂ is present at low concentrations (4-15% v/v) in gas mixtures near atmospheric pressure and at temperatures of approximately -60 °C. Sorbents for carbon capture are regenerated using temperature, pressure or vacuum, so that CO₂ can be collected for sequestration or utilization and the sorbent can be reused.

The most significant impediment to carbon capture is the large amount of electricity required. Without policy or tax incentives, the production of electricity from such plants is not competitive with other energy sources. The largest operating cost for power plants with carbon capture is the reduction in the amount of electricity produced,[6] because energy in the form of steam is diverted from making electricity in the turbines to regenerating the sorbent. Thus, minimizing the amount of energy required for sorbent regeneration is the primary goal behind much carbon capture research.

The present invention (Figure 2) follows a similar principle as for the standard solution mentioned above, but operating the concentration column at higher pressure, at least 2 barg but preferably between approximately 2 to 10 barg, in order to re-use the overhead duty. By operating the column at higher pressure, the boiling point of the liquids at various locations in the column increases. A higher temperature for the boiling liquid is targeted, between approximately 110 to 190 degrees Celsius. Higher temperature is required in the heat exchanger D, such as reboiler (imported steam) but the temperature for condensation in the overhead heat exchanger C, such as a reboiler, is also increased which allows that heat to be used elsewhere, as CO₂ reboiler duty or district heating.

Since green plants are small, typically only one concentration column exists in this type of plant and the duty cannot be reused in a second concentration column. Instead, the overhead duty is re-used at least partially in the CO₂ reboiler, so as to recycle such energy and minimize the waste.

### Drawings

Fig 1 shows the standard solution for production of methanol in a green methanol plant, with CO₂ stripping.
Fig. 2 shows a preferred embodiment of the present invention for production of methanol in a green methanol plant, with CO₂ stripping.

### Reference Numbers:

(1) - hot overhead stream comprising methanol vapor
(2) - heat provided from heat exchanger C to the CO₂ stripping unit A
(3) - steam stream

### Definitions

"Atmospheric pressure" means 1,01325 bar, i.e., approximately 1 bar.

Carbon capture means the method of capturing carbon dioxide from a stream, typically flue gas but also from pressurized process gas. The method consists of an absorber where a liquid sorbent is in contact with the gas and selectively absorbs the CO₂. The CO₂ loaded sorbent is sent to a stripper where the loaded CO₂ is stripped off by use of heat so that the CO₂ is leaving the stripper in concentrated form.

**Carbon dioxide sorbent** means a sorbent agent that is able to absorb CO₂. A carbon dioxide sorbent may be physical, such as a porous material or chemical, such as aqueous amine solutions that form a chemical bond.

**Carbon dioxide stripping unit** means a unit for desorption of the captured CO₂. Typically a column were loaded CO₂ solution is boiled to strip off the captured CO₂.

**Chemical sorbent** means a sorbent agent that absorbs CO₂ and forms a chemical bond with the active component and CO₂.

**"Concentration column"** or "distillation column" or "bottoming column" means a column divided into a series of stages. These correspond to a cascade of equilibrium stages. Liquid flows down the column from stage to stage and is contacted by vapor flowing upward. Traditionally, most columns have been built from a set of distinct "trays" or "plates", so these terms end up being essentially interchangeable with "stages". Each tray in a distillation column is designed to promote contact between the vapor and liquid on the stage. Distillation can be conducted in a packed column (just as absorption can be done in a trayed column). The operating pressure of the column is typically controlled by adjusting heat removal in the heat exchanger. The base of the column is typically used as a reservoir to hold liquid leaving the bottom tray. A heat exchanger, e.g. a reboiler, is used to boil this liquid. The vapor which results, the "boilup", is returned to the bottom of the column.

**Crude methanol** is distilled to meet the purity specifications required on the market. Crude methanol is a solution comprising methanol, typically 65 to 95% methanol, water and other components. Crude methanol contains low-boiling and high-boiling components (light and heavy ends). The light ends L include mainly dissolved gases (e.g., CO₂), di-methyl ether, methyl formate, and acetone. The heavy ends H include higher alcohols, long-chain hydrocarbons, higher ketones, and esters of lower alcohols with formic, acetic, and propionic acids. For example, the grade AA specification requires a minimum methanol concentration of 99.85% by weight, wherein ethanol should not exceed 10 ppm by weight.

"**Distillation**" means a method for separating liquid mixtures into two or more vapor or liquid products with different compositions. Distillation is an equilibrium stage operation. In each stage, a vapor phase is contacted with a liquid phase and mass is from vapor to liquid and from liquid to vapor. The less volatile, "heavy" or "high boiling", components concentrate in the liquid phase; the more volatile, "light", components concentrate in the vapor. By using multiple stages in series with recycle, separation can be accomplished. The feed to a distillation column may be liquid, vapor, or a liquid-vapor mixture. It may enter at any point in the column. More than one stream may be fed to the system, and more than one product may be drawn. Distillation at the concentration column typically takes place at the lowest possible pressure, but in the present invention we increase pressure to recover the heat.

**Green methanol plant** means a plant using renewable H₂ as feed.

"**Heat duty**" or "Duty" means the amount of heat needed to transfer from a hot side to the cold side over a unit of time. The equation to calculate the heat duty is normally written in two ways: a) one that can be used for sensible heat transferred, which means that the fluid undergoes no phase change; b) the other can be used for latent heat transferred, which means that the fluid undergoes a phase change. i.e. condenses.

"**Heat exchanger**" means a system used to transfer heat between two or more fluids. Heat exchangers are used in both cooling and heating processes. The fluids may be separated by a solid wall to prevent mixing or they may be in direct contact. In particular, "heat exchanger" means a reboiler/condenser, such as a tube bundle exchanger, for example with evaporation of the solution in the shell side and condensation of the distillate in the tube side (or vice-versa). It is also possible to use a plated heat exchanger with heat exchange plates housed inside a shell.

"**Heavy by-products**" or "side streams", H, means a stream comprising higher alcohols and other minor bi-products recovered from the last concentration column - typically taken out in between the feed tray and the bottom of the column. It is also known as "fusel oil" and comprises water, residual methanol (ca. 1 % of the total) and most of the by-products of the synthesis reaction. Said fusel oil has a certain heat value and is usually used as a fuel or feed to a synthesis gas generation section. Side streams of fusel oil can also be extracted, if suitable, from the intermediate distillation stages.

**High pressure distillation** means a distillation method that operates above the normal operating pressure. Typically methanol distillation is operated at low pressures to ease the separation of components, but high pressure distillation is operated at elevated pressures, i.e. higher than 2 barg.

**Loaded carbon dioxide sorbent** means a solution comprising the captured CO₂.

**Methanol (MeOH) synthesis gas** means syngas containing components for MeOH synthesis, a mixture of H₂, CO and CO₂ ( alternatively only H₂ and CO₂).

**Overhead duty** means the transferred heat that is achieved by condensing the methanol vapor in the top of the column, e.g, a concentration column.

**"Partial reboiler"** means a reboiler where only part of the liquid in the column base is vaporized. The vapor produced is returned to the column, and the liquid stream is removed as product or feed to an additional column . The compositions of these three streams are different. Partial reboilers also provide an ideal separation stage. Sidestream reboilers can be used, which draw liquid off a tray, heat it, and then return the vapor liquid mixture to the same or similar trays.

**Physical sorbent** means a sorbent that absorbs CO₂ without forming a chemical bond with the active component and CO₂.

**"Pressure",** P, means gauge pressure and is measured in bar(g). Gauge pressure is the pressure relative to atmospheric pressure and it is positive for pressures above atmospheric pressure, and negative for pressures below it. The difference between bar and bar(g) is the difference in the reference considered. Measurement of pressure is always taken against a reference and corresponds to the value obtained in a pressure measuring instrument. If the reference in the pressure measurement is vacuum we obtain absolute pressure and measure it in bar only. If the reference is atmospheric pressure then pressure is cited in bar(g).

**Raw methanol** product means a liquid product directly from synthesis in step (d) in the method of the present invention, mainly methanol but also water, byproducts and dissolved gasses.

**"Reboiler"** means a heat exchanger typically used to provide heat to the bottom of industrial distillation columns. Reboilers boil the liquid from the bottom of a distillation column to generate vapors which are returned to the column, to drive the distillation separation. The heat supplied to the column by the reboiler at the bottom of the column is removed by the condenser at the top of the column. Most reboilers are of the shell and tube heat exchanger type and normally steam is used as the heat source in such reboilers. However, other heat transfer fluids like hot synthesis gas, oil or Dowtherm (TM) may be used. Fuel-fired furnaces may also be used as reboilers in some cases.

**"Stabilizing column"** or Topping column or pre-run column is for separating the more volatile components from the heavier components, both contained in the crude product, such as crude methanol.

**"Volatile components"** or "volatile substances" means components or substances which vaporize readily at low temperatures. Volatility can also describe the tendency of a vapor to condense into a liquid or solid: less volatile substances will more readily condense from a vapor than highly volatile ones. ***Vapor pressure*** is a measurement of how readily a condensed phase forms a vapor at a given temperature. A substance enclosed in a sealed vessel initially at vacuum (no air inside) will quickly fill any empty space with vapor. After the system reaches equilibrium and no more vapor is formed, this vapor pressure can be measured. Increasing the temperature increases the amount of vapor that is formed and thus the vapor pressure. In a mixture, each substance contributes to the overall vapor pressure of the mixture, with more volatile compounds making a larger contribution. ***Boiling point*** is the temperature at which the vapor pressure of a liquid is equal to the surrounding pressure, causing the liquid to rapidly evaporate, or boil. It is closely related to vapor pressure, but is dependent on pressure. The normal boiling point is the boiling point at atmospheric pressure, but it can also be reported at higher and lower pressures.

"**Upper stream**" or "Top stream" means a stream obtained or recovered from the upper section of a column.

### Description

The present invention provides for a reduced overall energy consumption of the green plant (re-use of the distillation energy into the stabilizer column and CO₂ reboiler in carbon capture unit) and thereby a reduced electrical import for heater/boiler (or reduced fuel consumption in steam generation).

The present invention uses one or more columns for distillation, wherein the column at the highest pressure is connected to at least one CO₂ stripping unit. Said one or more columns comprise a stabilizing column V0, at pressure P0, connected in series with at least one distillation column V1, at pressure P1, wherein each column is associated to a heat exchanger E0 and E1, said heat exchanger being a reboiler for that column, characterized in that,
a) E1 has an incoming heat stream, external to said apparatus;
d) P1> 2 barg.

One purpose of the present invention is the reduction of energy input for green methanol plants. A standard layout has been developed where the plant consists of electrolyser, carbon capture, methanol synthesis and methanol distillation. Both a traditional carbon capture unit and the methanol distillation unit require heat in order to drive the CO₂ stripping (e.g., in a carbon capture unit) and the raw methanol distillation. Normally the heat is supplied by steam and since there is no excess steam available in a typical green methanol plant, this steam has to be generated by electricity (if CO₂ emissions should be minimized) or alternatively burning fuel to generate the steam.

By changing to a high pressure distillation step the overhead duty obtained from the overhead stream (1) in the concentration column can be used as reboiler duty in the CO₂ stripping process (in carbon capture unit) and alternatively also in the stabilizer column inside the methanol distillation or supplied to district heating. For the method of the invention, the operating pressure at the concentration column is higher than or approximately 2 barg.

The present invention has as main advantage the overall reduced energy consumption, i.e., approximately the same amount of heat sent to the concentration column can be reused in the CO₂ reboiler.

### Examples

### Example 1

### Energy consumption in CO₂ stripper and methanol distillation

**Table 1. Comparison of energy consumption in CO₂ stripper and methanol distillation for traditional layout versus new invention with re-use of energy from concentration column overhead MeOH vapor. Total steam import is reduced to 61% of the standard solution. In this case the re-use of heat is directed to the CO₂ stripper reboiler, as an alternative it could also substitute the steam in the stabilizer reboiler or alternatively used as district heating.**

| | Heating source | Unit | Standard Solution | Invention |
|---|---|---|---|---|
| CO2 stripper duty (steam) | Steam | MW | 8.7 | 0.6 |
| CO2 stripper duty (MeOH) | MeOH | MW | 0.0 | 8.1 |
| Stabilizer reboiler duty | Steam | MW | 1.2 | 1.2 |
| Concentration column duty | Steam | MW | 6.9 | 8.5 |
| **Total steam consumption** | | **MW** | **16.8** | **10.3** |
| **Total steam consumption** | | **%** | **100** | **61** |

By using the method, system and plant of the present invention, the total steam consumption is therefore significantly reduced, in this particular case by approximately 39%, in relation to the standard solution.

## Claims

1. Method for the preparation of methanol, comprising the steps of:
(a) Capturing carbon dioxide by means of a carbon dioxide sorbent and forming a loaded carbon dioxide sorbent;
(b) passing the loaded carbon dioxide sorbent to a carbon dioxide stripping unit A;
(c) providing a methanol synthesis gas comprising hydrogen and carbon dioxide;
(d) passing the methanol synthesis gas from step (c) to a methanol synthesis and forming a raw methanol product;
(e) purifying the raw methanol product in a distillation unit B comprising at least one distillation column at a pressure of at least 2 barg, obtaining a hot overhead stream (1) from said at least one distillation column, **characterized in that** said hot overhead stream (1) provides heat to heat exchanger C and at least part of said heat (2) is either provided to the, at least one, carbon dioxide stripping unit A for stripping carbon dioxide from the loaded carbon dioxide sorbent, thereby condensing stream (1) into liquid methanol, or is used as stabilizer reboiler duty or is directed to district heating,
wherein said heat (2) provided to at least one carbon dioxide stripping unit A, in particular to a reboiler D, said heat being approximately between 45% and up to 100% of the energy requirements in said stripping unit, optionally supplying additional heat with steam to reboiler D.

2. Method according to claim 1 wherein the methanol synthesis gas in step (c) comprises hydrogen partially or totally obtained from electrolysis.

3. Method according to claim 1 wherein the methanol synthesis gas in step c) comprises hydrogen obtained from sources other than electrolysis.

4. Method according to claim 1, wherein the carbon dioxide sorbent in step (a) is a physical or chemical sorbent.

5. Method according to claim 1, wherein the methanol synthesis gas further comprises carbon monoxide.

6. Method according to claim 1, wherein the carbon dioxide in step (a) originates from flue gas and/or synthesis gas.

7. Method according to claim 1, wherein part or all hot overhead stream (1) is supplied to district heating.

8. Method according to claims 1 or 7, wherein part of the hot overhead stream (1) is supplied to the stabilizing reboiler.

## Patentansprüche

1. Verfahren zur Herstellung von Methanol, umfassend die folgenden Schritte:
(a) Auffangen von Kohlendioxid mittels eines Kohlendioxid-Sorptionsmittels und Bilden eines beladenen Kohlendioxid-Sorptionsmittels;
(b) Weiterleiten des beladenen Kohlendioxid-Sorptionsmittels an eine Kohlendioxid-Stripping-Einheit A;
(c) Bereitstellen eines Methanolsynthesegases, das Wasserstoff und Kohlendioxid umfasst;
(d) Weiterleiten des Methanolsynthesegases aus Schritt (c) zu einer Methanolsynthese und Bilden eines Rohmethanolprodukts;
(e) Reinigen des Rohmethanolprodukts in einer Destillationsanlage B, die mindestens eine Destillationskolonne umfasst, bei einem Druck von mindestens 2 barg, Gewinnen eines heißen Kopfstroms (1) aus der mindestens einen Destillationskolonne, **dadurch gekennzeichnet, dass** der heiße Kopfstrom (1) Wärme an den Wärmetauscher C abgibt und mindestens ein Teil dieser Wärme (2) entweder der mindestens einen Kohlendioxid-Strippanlage A zur Abtrennung von Kohlendioxid vom beladenen Kohlendioxid-Sorptionsmittel zugeführt wird, wodurch der Strom (1) zu flüssigem Methanol kondensiert, oder als Stabilisator-Verdampferwärme verwendet oder der Fernwärme zugeführt wird,
wobei die Wärme (2) mindestens einer Kohlendioxid-Strippanlage A, insbesondere einem Verdampfer D, zugeführt wird, wobei die Wärme etwa zwischen 45 % und bis zu 100 % des Energiebedarfs der Stripanlage beträgt, wobei dem Verdampfer D optional zusätzliche Wärme mit Dampf zugeführt wird.

2. Verfahren nach Anspruch 1, wobei das Methanolsynthesegas in Schritt (c) teilweise oder vollständig durch Elektrolyse gewonnenen Wasserstoff umfasst.

3. Verfahren nach Anspruch 1, wobei das Methanolsynthesegas in Schritt c) Wasserstoff aus anderen Quellen als der Elektrolyse umfasst.

4. Verfahren nach Anspruch 1, wobei es sich bei dem Kohlendioxid-Sorptionsmittel in Schritt (a) um ein physikalisches oder chemisches Sorptionsmittel handelt.

5. Verfahren nach Anspruch 1, wobei das Methanolsynthesegas weiter Kohlenmonoxid umfasst.

6. Verfahren nach Anspruch 1, wobei das Kohlendioxid in Schritt (a) aus Rauschgas und/oder Synthesegas stammt.

7. Verfahren nach Anspruch 1, wobei ein Teil oder der gesamte heiße Kopfstrom (1) der Fernwärme zugeführt wird.

8. Verfahren nach Anspruch 1 oder 7, wobei ein Teil des heißen Kopfstroms (1) dem Stabilisierungsverdampfer zugeführt wird.

## Revendications

1. Procédé de préparation de méthanol, comprenant les étapes consistant à :
(a) capturer du dioxyde de carbone au moyen d'un sorbant de dioxyde de carbone et former un sorbant de dioxyde de carbone chargé ;
(b) faire passer le sorbant de dioxyde de carbone chargé vers une unité de désorption de dioxyde de carbone A ;
(c) fournir un gaz de synthèse de méthanol comprenant de l'hydrogène et du dioxyde de carbone ;
(d) faire passer le gaz de synthèse de méthanol de l'étape (c) à une synthèse de méthanol et former un produit de méthanol brut ;
(e) purifier le produit de méthanol brut dans une unité de distillation B comprenant au moins une colonne de distillation à une pression d'au moins 2 barg, obtenant un flux de tête chaud (1) à partir de ladite au moins une colonne de distillation, **caractérisé en ce que** ledit flux de tête chaud (1) fournit de la chaleur à l'échangeur de chaleur C et qu'au moins une partie de ladite chaleur (2) est soit fournie à la au moins une unité de désorption de dioxyde de carbone A pour désorber le dioxyde de carbone du sorbant de dioxyde de carbone chargé, condensant ainsi le flux (1) en méthanol liquide, soit utilisée pour alimenter le rebouilleur de stabilisateur ou est dirigée vers le chauffage urbain,
dans lequel ladite chaleur (2) fournie à au moins une unité de désorption de dioxyde de carbone A, en particulier à un rebouilleur D, ladite chaleur représentant approximativement entre 45 % et 100 % des besoins énergétiques de ladite unité de désorption, fournissant éventuellement une chaleur supplémentaire avec de la vapeur au rebouilleur D.

2. Procédé selon la revendication 1 dans lequel le gaz de synthèse de méthanol à l'étape (c) comprend de l'hydrogène partiellement ou totalement obtenu par électrolyse.

3. Procédé selon la revendication 1 dans lequel le gaz de synthèse de méthanol à l'étape c) comprend de l'hydrogène obtenu à partir de sources autres que l'électrolyse.

4. Procédé selon la revendication 1, dans lequel le sorbant de dioxyde de carbone de l'étape (a) est un sorbant physique ou chimique.

5. Procédé selon la revendication 1, dans lequel le gaz de synthèse de méthanol comprend en outre du monoxyde de carbone.

6. Procédé selon la revendication 1, dans lequel le dioxyde de carbone de l'étape (a) provient des gaz de combustion et/ou des gaz de synthèse.

7. Procédé selon la revendication 1, dans lequel une partie ou la totalité du flux de tête chaud (1) est fournie au chauffage urbain.

8. Procédé selon les revendications 1 ou 7, dans lequel une partie du flux de tête chaud (1) est fournie au rebouilleur de stabilisateur.
